# DEMANDE DE BREVET EUROPEEN

(11) **EP 0 572 344 A1**
(43) Date de publication de la demande: **01.12.1993**
(21) Numéro de dépôt: 93480062.4
(22) Date de dépôt: 24.05.1993
(51) Int. Cl.: C12M 1/20, C12Q 1/18

(54) **Disque pour l'étude de l'interaction de deux antibiotiques ou d'un antibiotique associé à un produit actif sur le métabolisme bactérien et son procédé de mise en oeuvre**

(30) Priorité: 27.05.1992 FR 9206681
(71) Demandeur: Drugeon, Henri, F-06500 Menton (FR)
(72) Inventeur: Drugeon, Henri, F-06500 Menton (FR)
(74) Mandataire: Hautier, Jean-Louis

(57) **Abrégé**

L'invention a pour objet un disque pour antibiotique.

Disque ou rondelle du type rond en papier filtre stérile utilisé de manière usuel pour les tests d'antibiotiques caractérisé par le fait qu'il comporte, en son centre, un trou (16) et qu'il est chargé avec l'un des deux produits (A, B) à étudier, ledit trou (16) est réalisé de manière à pouvoir recevoir le disque usuel d'antibiotique dit "disque complémentaire" qui vient prendre sa place dans ledit trou central (16) dudit disque percé (15).

L'invention s'applique à l'étude de l'interaction de deux antibiotiques.

## Description

L'invention a pour objet un disque pour l'étude de l'interaction de deux antibiotiques ou d'un antibiotique associé à un produit actif sur le métabolisme bactérien, par exemple un inhibiteur de beta-lactamase et son procédé de mise en oeuvre.

Deux techniques sont actuellement utilisées pour reconnaître l'activité antibiotique d'une substance chimique :
- en premier, la technique de dilution en milieu liquide permet de rechercher, par des dilutions successives du produit à expérimenter, la concentration minimale inhibitrice (C.M.I.)
- en second, la technique de la diffusion en gélose ou méthode des disques.

A la surface d'un milieu gélosé contenu dans des boîtes de Pétri, après ensemencement par le germe à étudier, on applique des rondelles ou disques de papier filtre stériles chargés ou imprégnés des antibiotiques à tester, à des concentrations différentes d'un disque à l'autre. Les antibiotiques diffusent radialement dans la gélose à partir des disques, de telle sorte qu'autour de chacun d'eux s'établit un gradient de concentration décroissant avec la distance par rapport au disque. Après quelques heures d'étuve, les disques apparaissent entourés d'une zone d'inhibition dont le diamètre permet de calculer la C.M.I. comparativement à un étalonnage effectué avec des antibiotiques connus.

Dans différentes circonstances, il est nécessaire de charger le disque avec deux antibiotiques ou avec un antibiotique associé à un produit actif sur le métabolisme bactérien par exemple : un inhibiteur de béta-lactamase.

*L'état de la technique peut être défini par les brevets* *suivants :*
- *FR-A-2.288.782 : l'invention décrit un support en forme* *d'étoile à six branches dont chaque extrémité porte un disque de* *test.*
   *Le corps de base comporte une zone centrale entourée de six* *bras équidistants. Leurs extrémités comportent des surfaces pour* *la fixation des disques de test. La zone centrale comporte deux* *évidements pour la saisie du corps à l'aide de brucelles. La* *partie des bras est en profilé creux. A part les extrémités* *portant les disques, tout le support est au-dessus du plan des* *disques.*
- *FR-A-2.281.297 : ce distributeur est caractérisé par un* *mécanisme de table de distribution comportant des trous supérieurs* *et inférieurs décalés ; un moyen de support de cartouches de* *disques, une plaque de distribution à ouverture permettant* *d'aligner les trous supérieurs et inférieurs, et des ouvertures* *auxiliaires dans le mécanisme de distribution de table permettant* *de contrôler visuellement le fonctionnement du distributeur et de* *dégager éventuellement les disques coincés.*
- *FR-A-2.268.076 : on prépare des disques d'essai de* *sensibilité aux antibiotiques en mettant un matériau absorbant en* *contact avec une dispersion d'un antibiotique du type béta-lactame* *cristallisé dans un véhicule non solvant volatil, en faisant* *absorber cette dispersion par ce matériau, en évaporant ce* *véhicule de ce matériau, ce qui laisse un dépôt uniforme* *d'antibiotique par unité de surface de matériau. On peut ensuite* *découper des disques normalisés d'essai de sensibilité aux* *antibiotiques.*

Ces brevets et beaucoup d'autres traitent essentiellement de la distribution des disques.

Dans le cas où il est nécessaire de charger le disque avec deux antibiotiques ou avec un antibiotique associé à un produit actif sur le métabolisme bactérien, il est possible de préparer un disque contenant les deux produits, mais ceci est difficile à mettre en place dans la routine quotidienne d'un laboratoire d'analyse médicale. De plus, si l'on doit faire varier considérablement les antibiotiques associés, il est nécessaire de posséder un très grand nombre de disques différents correspondant aux différentes associations à étudier.

A cet effet, l'invention a pour objet un disque dont la caractéristique technique permet le mariage de différentes molécules deux à deux. Il est fondé sur le fait que la diffusion est radiale.

L'invention a également pour objet le procédé de mise en oeuvre.

Le disque ou rondelle selon l'invention est du type rond en papier filtre stérile utilisé de manière usuel pour les tests d'antibiotiques, ledit disque est caractérisé par le fait qu'il comporte, en son centre, un trou et qu'il est chargé avec l'un des deux produits à étudier, ledit trou est réalisé de manière à pouvoir recevoir le disque usuel d'antibiotique dit "disque complémentaire" qui vient prendre sa place dans ledit trou central.

Selon un mode de réalisation particulier, le diamètre du disque selon l'invention est d'environ 11,5 mm, le diamètre du trou est de 7 mm.

Les dessins ci-joints sont donnés à titre d'exemples indicatifs et non limitatifs. Ils représentent un mode de réalisation préféré selon l'invention. Ils permettront de comprendre aisément l'invention.

La figure 1 est une vue de la technique des bandelettes.

La figure 2 est une vue de la technique des disques alignés.

La figure 3 est une vue de la technique des deux disques à la suite.

La figure 4 est une vue de la technique du double disque superposé.

La figure 5 est une vue de la technique d'incorporation à la gélose d'un des deux antibiotiques.

La figure 6 est une vue en plan du disque percé d'un trou selon l'invention.

La figure 7 est une vue d'un disque du type usuel.

La figure 8 est une vue en plan du disque percé qui a reçu le disque usuel dans le trou prévu à cet effet.

La figure 9 met en évidence le procédé selon l'invention avec une vue en plan des pastilles disposées dans une boîte de Pétri.

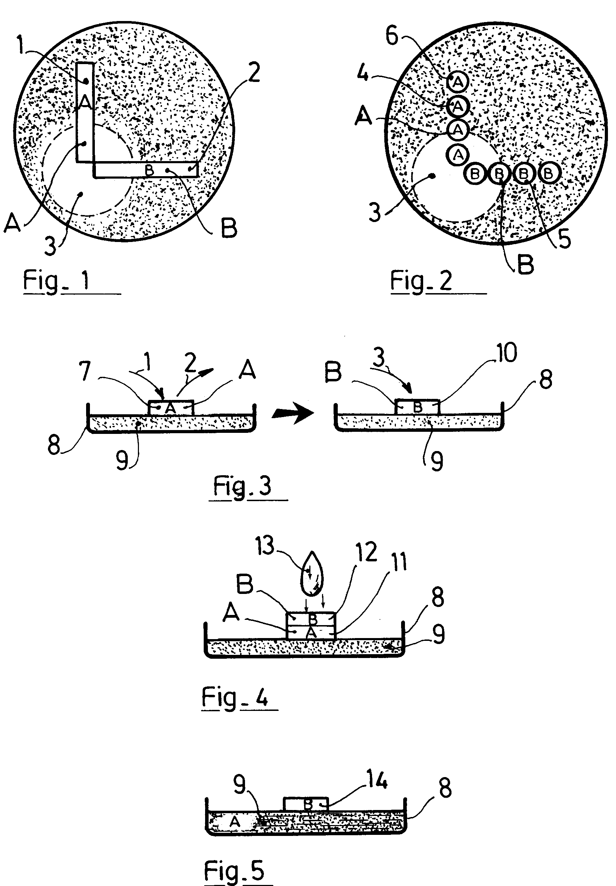

La technique, représentée à la figure 1, utilise deux bandelettes 1, 2 qui sont chacune chargée d'un produit A ou B. Au niveau de la fonction des deux bandelettes 1, 2, une zone d'interaction 3 se crée mais elle est très délicate à interpréter et impossible à étudier en routine.

La technique des disques alignés est quasiment identique à la technique des bandelettes, si ce n'est que les bandelettes sont remplacées par deux séries 4, 5 de disques usuels 6. Une série 4 de disques usuels 6 est chargée en produit A, une seconde série 5 de disques usuels 5 est chargée en produit B.

La zone d'interaction 3 présente les mêmes inconvénients que la technique précédente.

La technique des deux disques à la suite est représentée à la figure 3.

Il est mis un disque usuel 7 chargé en produit A dans la boîte de Petri 8 contenant de la gélose 9, puis on place un autre disque usuel 10 chargé en produit B au même endroit.

La technique du double disque est représentée à la figure 4.

Les deux disques usuels 11 et 12 sont superposés, l'un le disque 11 est chargé en produit A, l'autre le disque 12 est chargé en produit B. Une goutte d'eau 13 est posée sur le disque supérieur 12 pour mélanger les produits A, B des deux disques 11 et 12. Puis, l'utilisateur enlève le disque 12 deux heures après.

Une autre technique, représentée à la figure 5, consiste à incorporer à la gélose 9 le produit A et à disposer, sur la gélose, le disque usuel 14 chargé en produit B.

Le disque 15 selon l'invention est du type rond en papier filtre stérile utilisé de manière usuelle pour les tests d'antibiotiques.

Le disque 15 est caractérisé par le fait qu'il comporte, en son centre, un trou 16.

Le trou 16 est réalisé de manière à pouvoir recevoir un disque usuel 17.

Le disque 15 a un diamètre d'environ 11,5 mm.

Le trou 16, percé en son centre, a un diamètre d'environ 7 mm.

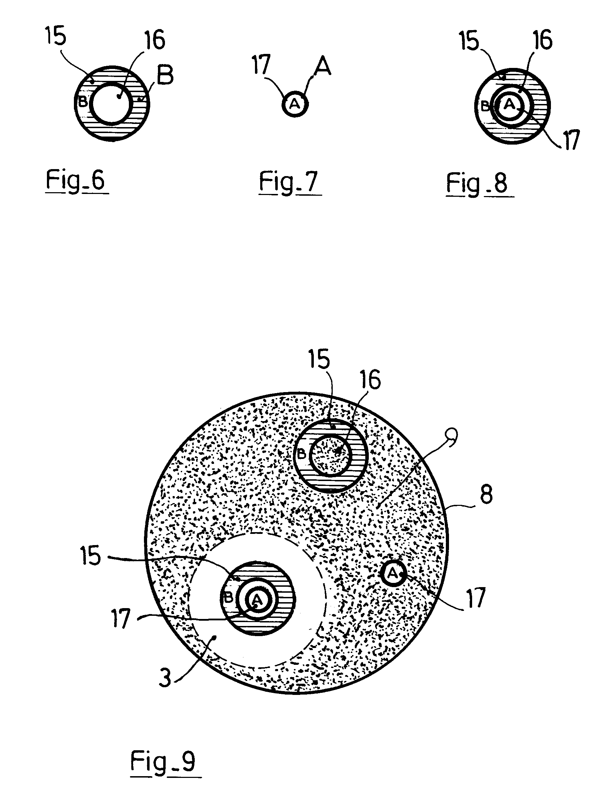

Selon le procédé de mise en oeuvre représenté à la figure 9, le disque percé 15 est chargé avec l'un des deux produits à étudier selon la figure 8, le produit B. Pour l'autre produit A, on utilise le disque usuel 17 d'antibiotique dont le diamètre est de 6,5 mm et qui vient prendre place dans le trou central du disque 15. Pour les deux produits A et B, le gradient de concentration radiale s'établit.

La lecture des résultats s'effectue comme pour l'antibiogramme classique en mesurant les diamètres d'inhibition et en les comparant soit à ceux obtenus à partir des disques des produits isolés, soit aux diamètres critiques déterminés par les comités de l'antibiogramme : CFA (Comité Français d'Antibiogramme), NCCLS (National Comity Central Laboratory S....), OMS (Office Mondial de la Santé).

Le diamètre critique déterminant la résistance bactérienne est au minimum de 13 mm ; c'est pourquoi le diamètre du disque fait 11,5 mm. Il doit être suffisamment important pour permettre une rigidité du disque et suffisamment petit pour permettre la lecture de la zone de résistance.

### REFERENCES

1, 2. Bandelettes
3. Zone d'interaction
4, 5. Séries de disques usuels
6. Disques usuels
7. Disque usuel chargé en produit A
8. Boîte de Petri
9. Gélose
10. Disque usuel chargé en produit B
11. Disque usuel chargé en produit A
12. Disque usuel chargé en produit B
13. Goutte d'eau
14. Disque usuel chargé en produit B
15. Disque
16. Trou
17. Disque usuel chargé en produit A A, B Produits

## Revendications

1. Disque ou rondelle du type rond en papier filtre stérile utilisé de manière usuel pour les tests d'antibiotiques caractérisé par le fait
qu'il comporte, en son centre, un trou (16) et qu'il est chargé avec l'un des deux produits (A, B) à étudier, ledit trou (16) est réalisé de manière à pouvoir recevoir le disque usuel d'antibiotique dit "disque complémentaire" qui vient prendre sa place dans ledit trou central (16) dudit disque percé (15).

2. Disque ou rondelle selon la revendication 1 caractérisé par le fait
que le diamètre du disque percé (15) est d'environ 11,5 mm, le diamètre du trou (16) est de 7 mm.

3. Disque ou rondelle selon la revendication 1 caractérisé par le fait
que le diamètre critique déterminant la résistance bactérienne est au minimum de 13 mm ; le diamètre du disque (15) fait 11,5 mm ; il doit être suffisamment important pour permettre une rigidité du disque (15) et suffisamment petit pour permettre la lecture de la zone de résistance.

4. Procédé de mise en oeuvre selon l'une quelconque des revendications 1, 2 ou 3 caractérisé par le fait
que le disque percé (15) est chargé avec l'un des deux produits, le produit (B) ; pour l'autre produit (A), on utilise le disque usuel (17) d'antibiotique qui vient prendre place dans le trou central (16) du disque percé (15) ; pour les deux produits (A) et (B), le gradient de concentration radiale s'établit.
